# EUROPÄISCHE PATENTANMELDUNG

(11) **EP 2 607 346 A1**
(43) Veröffentlichungstag der Anmeldung: **26.06.2013**
(21) Anmeldenummer: 11194982.2
(22) Anmeldetag: 21.12.2011
(51) Int. Cl.: C07C 51/573, C07C 57/10

(54) **Verfahren zur Herstellung, Stabilisierung und Konfektionierung von Sorbinsäureanhydrid**

(71) Anmelder: Saltigo GmbH, 40764 Langenfeld (DE)
(72) Erfinder: Krahwinkel, Ralf, 40764 Langenfeld (DE); Markert, Robert, 51377 Leverkusen (DE)
(74) Vertreter: Pettrich, Klaus-Günter

(57) **Zusammenfassung**

Die vorliegende Erfindung betrifft ein Verfahren zur Herstellung von Sorbinsäureanhydrid, vorzugsweise in Form des all-E-Isomers, mit einem möglichst geringen Gehalt an Lösungsmittel und die Vermeidung der Verringerung des Sorbinsäureanhydridgehalts bei der weiteren Handhabung durch Konfektionierung in geeignete Anwendungsformen, welche keines Aufschmelzens bedürfen, oder durch Zugabe eines geeigneten Stabilisators.

## Beschreibung

Gegenstand der Erfindung ist ein Verfahren zur Herstellung von Sorbinsäureanhydrid, insbesondere von all-E-Sorbinsäureanhydrid in hoher Isomerenreinheit, aus Mischungen enthaltend Lösungsmittel und Sorbinsäureanhydrid, sowie die Stabilisierung des Sorbinsäureanhydrids und die Konfektionierung in Formen mit verbesserter Handhabbarkeit.

Die Herstellung von Sorbinsäureanhydrid per se ist bekannt und u. a. in DE1283823A, GB1138813A, CN101357884A und Eckart, Cotarca, "Phosgenations: A Handbook", Wiley-VCH 2003, p.353-357 beschrieben. Die jeweiligen Verfahren sind jedoch mit den Nachteilen mangelnder Isomerenreinheit oder mangelnder Produktstabilität behaftet. In der noch unveröffentlichten Patentanmeldung PCT/EP2011/068860 wird ferner ein Syntheseweg beschrieben, bei dem Alkali-oder Erdalkalisalze der Sorbinsäure in einem inerten organischen Lösungsmittel mit Phosgen umsetzt werden. Mit diesem Verfahren kann Sorbinsäureanhydrid in hoher Isomerenreinheit als all-E-Isomer erhalten werden, welches, wie aus DE-A-102006035202 und WO 2008/014889 bekannt, eine besonders ausgeprägte konservierende Wirkung erzielt. Für verschiedene Anwendungen, speziell für die Verwendung als Konservierungsmittel in Nahrungsmitteln wird ein geringer Restlösungsmittelgehalt von weniger als 1000 ppm insbesondere weniger als 500 ppm gefordert: Mit den oben genannten Verfahren ist es jedoch nicht möglich, das Sorbinsäureanhydrid ausreichend vom Lösungsmittel zu befreien, ohne dass dabei eine Verschlechterung der Produktqualität durch Polymerisation, Isomerisierung bzw. Oxidation auftritt.

Dies beruht darauf, dass bereits ab 60 °C die exotherme Zersetzung des Sorbinsäureanhydrids beginnt, so dass wegen der thermischen Belastung des Produkts einerseits und aus Sicherheitsgründen wegen der relativ hohen Zersetzungsenthalpie von ca. 1100 J/g andererseits, ein ansatzweises (Batch-)destillationsverfahren nicht durchgeführt werden kann. Somit ist bei einer Reinigung im technischen Maßstab die Verwendung eines kontinuierlichen Verfahrens, z. B. mittels eines Dünnschichtverdampfers erforderlich, wobei aufgrund der thermischen Labilität des Sorbinsäureanhydrids auch hiermit das Lösungsmittel nicht ausreichend abgetrennt werden kann.

Weiterhin gestaltet sich die Handhabung des chemisch und thermisch empfindlichen Sorbinsäureanhydrids schwierig, da trotz der relativ niedrigen Schmelztemperatur von ca. 31 °C (all-E-Isomer) bei wiederholtem Aufschmelzen rasch eine Gelierung auftritt und der Sorbinsäureanhydridgehalt deutlich abnimmt. Eine Lagerung in flüssiger Form, insbesondere bei erhöhter Lagerungstemperatur ist jedoch auch nicht praktikabel, da hierbei ebenfalls eine Verringerung des Sorbinsäuregehalts erfolgt. Wenn das hergestellte Sorbinsäureanhydrid in Form von Blöcken erstarrt und kühl gelagert wird, so ist dies zwar vorteilhaft für die Lagerung, jedoch wird die Zerkleinerung sehr aufwändig und die Zudosierung, sowie eine Vermischung mit bzw. Lösung in einer anderen Substanz erschwert. Eine Reinigung und Konfektionierung durch Kristallisation wiederum scheitert an geeigneten Lösungsmitteln, da das Sorbinsäureanhydrid in unpolaren Lösungsmitteln gut löslich ist und praktisch nicht mehr ausfällt. In polaren protischen Lösungsmitteln ist Sorbinsäureanhydrid hingegen zwar relativ schlecht löslich, wird aber durch diese Lösungsmittel chemisch verändert bzw. durch deren Restfeuchtigkeit partiell zersetzt.

Somit bestand die Aufgabe der vorliegenden Erfindung darin, ein Verfahren bereitzustellen, durch welches Sorbinsäureanhydrid mit einem möglichst geringen Gehalt an Lösungsmittel erhalten wird, ohne dass eine Polymerisierung bzw. Isomerisierung des Sorbinsäureanhydrids in nennenswertem Umfang auftritt. Zudem sollten Verfahren gefunden werden, durch welche Sorbinsäureanhydrid gegen Zersetzung stabilisiert wird bzw. in leichter handhabbare Anwendungsformen konfektioniert werden kann.

Allgemein wird in der verfahrenstechnischen Lehre davon ausgegangen, dass man Leichtsieder nur dann vollständig entfernen kann, wenn bis zum Siedepunkt der nächst höhersiedenden Komponente destilliert wird. Im Falle von Sorbinsäureanhydrid als nächst höhersiedender Komponente ist jedoch gerade dies aufgrund der thermischen Labilität des Stoffes nicht möglich.

Überraschend wurde nun gefunden, dass dennoch ein besonders lösungsmittelarmes Sorbinsäureanhydrid erhalten werden kann, indem man aus einer Mischung enthaltend Sorbinsäureanhydrid und Lösungsmittel das Lösungsmittel mittels eines Verdampfers zumindest teilweise entfernt, wobei der Verdampfer im kontinuierlichen Verfahren so betrieben wird, dass die Mischung enthaltend Sorbinsäureanhydrid und Lösungsmittel mit einem Inertgasstrom, bevorzugt einem Stickstoffstrom in Kontakt gebracht wird.

Die Menge an Inertgas liegt dabei üblicherweise in einem Bereich von 0,1 und 10000, vorzugsweise von 1 und 1000 und besonders bevorzugt von 10 und 100 Norm 1 pro kg Mischung.

Unter Lösungsmittel im Sinne der Erfindung sind insbesondere aprotische organische Lösungsmittel mit einem Siedepunkt von unter 145 °C bei 1013 mbar zu verstehen. Die Lösungsmittel können aus einem einzelnen Stoff oder einer Mischung von Stoffen bestehen. Bevorzugt handelt es sich beim Lösungsmittel um Toluol, Xylol, Chlorbenzol, Methyl-tert.-butylether, Isoamylacetat, Butylacetat, Ethylacetat, Methylacetat oder beliebige Mischungen davon, wobei Ethylacetat besonders bevorzugt ist.

Erfindungsgemäß ist unter Inertgas ein gasförmiges Medium zu verstehen, welches unter den jeweils angewandten Verfahrensbedingungen nicht oder nur in einem unwesentlichen Umfang mit dem Sorbinsäureanhydrid reagiert. Unwesentlich bedeutet in diesem Zusammenhang, dass weniger als 5 Gew.-%, bevorzugt weniger als 1 Gew.-% besonders bevorzugt weniger als 0,1 Gew.-% des Sorbinsäureanhydrids mit dem Inertgas reagieren. Bevorzugte Inertgase sind Luft mit einem H₂O-Partialdruck von weniger als 2 %, bevorzugt weniger als 1 %, besonders bevorzugt weniger als 0,1 % und ganz besonders bevorzugt weniger als 0,01% des Gesamtdrucks der Luft, sowie Stickstoff, Wasserstoff, Kohlendioxid, Helium, Neon, Krypton oder Argon, weiterhin Methan, Ethan oder Propan, welche mit Fluor, Chlor und/oder Brom substituiert sein können. Diese Gase können sowohl einzeln als auch in beliebigen Mischungen derselben eingesetzt werden.

Der Verdampfer kann vom Fachmann anhand der Fachliteratur für Verfahrenstechnik geeignet gewählt werden. Bevorzugt wird ein Fallfilm- oder Dünnschichtverdampfer, besonders bevorzugt ein Dünnschichtverdampfer verwendet. Der Verdampfer wird bei einem Druck von unter 1013 mbar, vorzugsweise unter 500 mbar, besonders bevorzugt unter 200 mbar und ganz besonders bevorzugt bei 100mbar oder weniger betrieben.

Das erfindungsgemäße Verfahren ermöglicht es, Sorbinsäureanhydrid mit einem Gehalt an Lösungsmittel von 1000 ppm oder weniger, bevorzugt von 500 ppm oder weniger und besonders bevorzugt von 200 ppm oder weniger zu erhalten.

In einer bevorzugten Ausführungsform wird durch das erfindungsgemäße Verfahren all-E-Sorbinsäureanhydrid in hoher Isomerenreinheit enthalten, d.h. mehr als 96%, bevorzugt mehr als 98%, besonders bevorzugt mehr als 99% und ganz besonders bevorzugt mehr als 99,5 % bezogen auf den Gesamtgehalt an Sorbinsäureanhydrid liegen als all-E-Sorbinsäureanhydrid vor.

In einer Ausführungsform umfasst das erfindungsgemäße Verfahren die Herstellung der Mischung aus Sorbinsäureanhydrid und Lösungsmittel durch den Schritt des Inkontaktbringens von einem oder mehreren Alkali- oder Erdalkalisalzen der Sorbinsäure mit Phosgen in einem geeigneten Lösungsmittel. Diese Verfahrensweise bietet neben der hohen erzielbaren Isomerenreinheit den Vorteil, dass die Mischung enthaltend Sorbinsäureanhydrid und Lösungsmittel nur geringe Mengen an Verunreinigungen neben Sorbinsäure aufweist, so dass nach Abtrennung des Lösungsmittels fast ausschließlich Sorbinsäureanhydrid und ein geringer Anteil von unter 5 Gew.-%, vorzugsweise unter 3 Gew.-% und besonders bevorzugt von unter 2 Gew.-% Sorbinsäure erhalten wird, was für die Anwendung als Konservierungsmittel vorteilhaft ist. Das vorliegende Verfahren kann auch derart geführt werden, dass bereits vor dem Einbringen in den Verdampfer ein Teil des für die Reaktion oder einen Aufarbeitungsschritt benötigten Lösungsmittels abgetrennt wird.

Falls eine besonders hohe Produktreinheit gefordert ist, kann das vorliegende Verfahren dadurch ergänzt werden, dass sich an die Entfernung des Lösungsmittels im Verdampfer eine Feinreinigung des Produkts mittels Kurzwegdestillation anschließt, wobei ein besonders reines und farbloses Sorbinsäureanhydrid erhalten werden kann, bei jedoch gleichzeitiger Verringerung der Ausbeute. Die Kurzwegdestillation wird typischerweise bei einem Druck von unter 20 mbar, vorzugsweise unter 5 mbar und besonders bevorzugt bei unter 0.5 mbar durchgeführt.

Überraschenderweise wurde festgestellt, dass die signifikante Abnahme des Sorbinsäureanhydridgehalts durch polymerisationsbedingte Gelierung bei der Lagerung bzw. bei, zur Portionierung des Stoffes erforderlichem, ggf. wiederholtem Aufschmelzen des festen Sorbinsäureanhydrids unterdrückt werden kann, indem vor oder nach der Entfernung des Lösungsmittels ein Antioxidans als Stabilisator zugegeben wird. Als Stabilisatoren können beispielsweise 2,4-Dimethyl-6-tert.-butylphenol, 2,6-Di-tert.-butyl-parakresol (BHT), Tocopherol(e) und Ascorbinsäure allein oder als beliebige Mischung davon verwendet werden. Eine Zugabe vor der Entfernung des Lösungsmittels ist verfahrenstechnisch einfacher zu realisieren, wodurch überraschenderweise trotz thermischer Behandlung ein lager- und aufschmelzstabilisiertes Sorbinsäureanhydrid erhalten werden konnte.

In einer weiteren bevorzugten Ausführungsform wird das erfindungsgemäße Verfahren so geführt, dass das Sorbinsäureanhydrid flüssig erhalten und auf einer gekühlten Oberfläche, bevorzugt auf einem Kälteband oder einer gekühlten Schuppenwalze kristallisiert wird. Geeignete Apparaturen sind beispielsweise in DE 19630532A1, EP 0459328A1, DE 60103866T2 beschrieben. Durch geeignetes Aufbringen auf die Oberfläche, beispielsweise durch Tropfen kann das Sorbinsäureanhydrid als Teilchen, beispielsweise in Form von Schuppen oder Pellets, mit einer räumlichen Ausdehnung in jede Dimension von 1 bis 20 mm, bevorzugt 1 bis 10 mm und besonders bevorzugt von 1 bis 6 mm erhalten werden, welche die weitere Handhabung des Sorbinsäureanhydrids bedeutend erleichtern. Die Kristallisation erfolgt vorzugsweise unter Beaufschlagung mit einem oder mehreren der oben beschriebenen Inertgase. Wenn ein besonders feinteiliges Sorbinsäureanhydrid gewünscht wird, so können die erhaltenen Schuppen oder Pellets bei einer Temperatur von weniger als 20 °C, bevorzugt von weniger als 10 °C und besonders bevorzugt von weniger als 0 °C zu einem Pulver vermahlen werden. Geeignete kühlbare Mühlen sind beispielsweise in DE 102010001861A1 und DE10163598A1 beschrieben. Durch die genannten Verfahrensweisen kann leicht handhabbares Sorbinsäureanhydrid in fester Form erhalten werden, das keines Aufschmelzens mehr bedarf, weshalb auf die Zugabe von Antioxidantien als Stabilisatoren verzichtet werden kann. Dies ist für verschiedene Verwendungszwecke und speziell beim Einsatz von Sorbinsäureanhydrid als Konservierungsstoff für Lebensmittel von Vorteil.

Ein weiterer Gegenstand der vorliegenden Erfindung ist Sorbinsäureanhydrid dadurch gekennzeichnet, dass der Gehalt an organischem Lösungsmittel 1000 ppm oder weniger, bevorzugt 500 ppm oder weniger und besonders bevorzugt 200 ppm oder weniger beträgt.

In einer bevorzugten Ausführungsform liegt das Sorbinsäureanhydrid zu mehr als 96%, bevorzugt zu mehr als 98%, besonders bevorzugt zu mehr als 99% und ganz besonders bevorzugt zu mehr als 99,5 % bezogen auf den Gesamtgehalt an Sorbinsäureanhydrid als all-E-Sorbinsäureanhydrid vor.

In einer weiteren Ausführungsform liegt das Sorbinsäureanhydrid als Mischung mit einem oder mehreren der obengenannten Stabilisatoren vor.

Weiterhin betrifft die vorliegende Erfindung ein Verfahren zur Konservierung von Lebensmitteln, enthaltend zumindest die Schritte eines der zuvor vorgestellten erfindungsgemäßen Verfahren zur Herstellung von Sorbinsäureanhydrid sowie die Kontaktierung des Lebensmittels mit dem Sorbinsäureanhydrid.

### Beispiel 1

### Dünnschichtdestillation von Sorbinsäureanhydrid mit Transportgasstrom

In einen handelsüblichen Labordünnschichtverdampfer aus Glas mit einer Verdampferfläche von 0,045 m², einer Manteltemperatur von 90°C und bei einem Druck von 100 mbar wurde 300 g/h einer Lösung enthaltend 20 Gew.-% Sorbinsäureanhydrid (davon 1,5 bis 2,5 Gew.-% Sorbinsäure) [Sorbinsäureanhydrid lag dabei zu ca. 99.5% als all-E Isomer vor] in Ethylacetat eingespeist und gleichzeitig am Dünnschichtverdampferfuß 20 Norm 1/h N₂ als Transportgasstrom eingeleitet.

Als Sumpfprodukt im Ablauf des Dünnschichtverdampfers wurde Sorbinsäureanhydrid in einer Reinheit von > 96 % (GC, Flammenionendetektor (FID)) [Sorbinsäureanhydrid lag dabei zu ca. 99.5% als all-E Isomer vor] und einem Restgehalt von < 500 ppm Ethylacetat erhalten. Der Sorbinsäuregehalt betrug 1,5-2,5 %. Das Produkt wies nach dem Erstarren in der Vorlage eine hellbeige Farbe auf. Die Destillationsausbeute betrug 96,1 %.

Vergleichsbeispiel 1: Dünnschichtdestillation von Sorbinsäureanhydrid ohne Transportgasstrom Unter analogen Bedingungen wie im obigen Beispiel 1 wurden Sorbinsäureanhydrid/Ethylacetatlösung in den Dünnschichtverdampfer eingespeist, wobei jedoch von einer Einleitung von N₂ als Transportgasstrom abgesehen wurde.

Im Ablauf des Dünnschichtverdampfers wurde Sorbinsäureanhydrid als Sumpfprodukt mit einem Gehalt von > 96 % (GC, FID) [[Sorbinsäureanhydrid lag dabei zu ca. 99.5% als all-E Isomer vor] und einem Restgehalt von > 1000 ppm Ethylacetat erhalten. Der Sorbinsäuregehalt betrug 1,5-2,5 %. Das Produkt wies nach dem Erstarren in der Vorlage eine hellbeige Farbe auf. Die Destillationsausbeute betrug > 96 %.

### Beispiel 2

### Kurzwegdestillation von Sorbinsäureanhydrid

82 g/h des als Sumpfprodukt erhaltenen Sorbinsäureanhydrids gemäß Beispiel 1 wurden in geschmolzener Form kontinuierlich in eine Kurzwegdestillationsapparatur eingebracht, und bei 110°C Manteltemperatur und einem Druck von 0,37 mbar verdampft, an einem auf 40°C temperierten Kühlfinger kondensiert und in die Destillationsvorlage geleitet.

Als Destillat wurde sehr reines Sorbinsäureanhydrid mit einem Gehalt von > 97% (GC, FID) [Sorbinsäureanhydrid lag dabei zu ca. 99.5% als all-E Isomer vor] und einem Restgehalt von < 500 ppm Ethylacetat erhalten. Der Sorbinsäuregehalt betrug ca. 1-1,5%. Das Produkt hatte nach dem Erstarren in der Vorlage eine rein weiße Farbe. Die Destillationsausbeute betrug 72,4 % d. Theorie. Der dunkle Sumpfrückstand der Kurzwegdestillation bestand zu 93,7 % aus Sorbinsäureanhydrid und ca. 1 % Sorbinsäure sowie ca. 5% unbekannten polymeren Crackprodukten.

### Beispiel 3

### Thermisches Verhalten und Alterung von unstabilisiertem Sorbinsäureanhydrid

Ein Teil des im Beispiel 1 erhaltenen Sorbinsäureanhydrids wurde unter Stickstoff bei 50°C aufgeschmolzen und dann 250 h bei dieser Temperatur gehalten. Der Gehalt an Sorbinsäureanhydrid wurde mittels GC verfolgt und verringerte sich dabei von anfangs ca. 96 % auf ca. 37 %. In einer ähnlichen Versuchsreihe konnte zudem ermittelt werden, dass beim wiederholten Aufschmelzen von Sorbinsäureanhydrid sehr schnell ein unlöslicher Bodensatz verbleibt und der Feststoffgehalt mit jedem Aufschmelzvorgang zunimmt und letztlich eine Abnahme des Sorbinsäureanhydrid gehalts in der überstehenden Schmelze und Gelierung eintritt.

### Vergleichsbeispiel 3

### Thermisches Verhalten und Alterung eines mit Stabilisator behandelten Sorbinsäureanhydrids

Die Versuche gemäß Beispiel 3 wurden wiederholt mit einem Sorbinsäureanhydrid, welchem vor dem Entfernen des Lösungsmittels im Verdampfer gemäß Beispiel 1, 0,1% BHT bezogen auf den Wirkstoffgehalt zugesetzt hat. Dabei zeigte sich, dass das stabilisierte Sorbinsäureanhydrid die Lagerung bei erhöhter Temperatur und die wiederholten Aufschmelzvorgänge wie in Beispiel 2 beschrieben erträgt ohne einen signifikanten Rückgang des Sorbinsäureanhydridgehalts zu erleiden.

So wurde eine Probe Sorbinsäureanhydrid unter den Bedingungen des Beispiels 3 gelagert und der Gehalt in regelmäßigen Abständen mittels GC kontrolliert. Es zeigt sich eine Abnahme des Gehalts von 96,5% auf 94 % nach 250 h; Gelierung oder Feststoffabscheidungen aus der Schmelze wurden nicht beobachtet.

### Beispiel 4

### Konfektionierung von Sorbinsäureanhydrid

Der Sumpfaustrag des Dünnschichtverdampfers bzw. das Destillat des Kurzwegverdampfers wurden unter Inertgas (N₂) direkt auf gekühlte Bleche bzw. auf eine Schuppenwalze aufgetragen. Dabei entstanden je nach Dosiergeschwindigkeit des Sorbinsäureanhydrids mehr oder weniger grobe Agglomerate (Schuppen oder Pellets) die sofort danach vom Blech oder der Walze abgeschält wurden. Die Schuppen oder Pellets wurden in einer handelsüblichen Kryo-Mühle (z.B. Fa. Retsch) bei -5 bis 0°C vermahlen und dadurch wenig kompaktierende Pulver von Sorbinsäureanhydrid erhalten. Die Pulver waren leicht portionier- und dosierbar und wurden unter inerten, dunklen und kühlen (< 0°C) Bedingungen gelagert.

Das Sorbinsäureanhydrid aus dem Sumpfaustrag des Dünnschichtverdampfers wurde dabei als hellbeiges Pulver mit einem Gehalt von > 96 % Sorbinsäureanhydrid, 1,5-2,5 % Sorbinsäure und < 500 ppm Ethylacetat erhalten.

Das Sorbinsäureanhydrid aus dem Destillat des Kurzwegverdampfers wurde als rein weißes Pulver mit einem Gehalt von >97% Sorbinsäureanhydrid, 1-1,5% Sorbinsäure und < 500 ppm Ethylacetat erhalten.

## Patentansprüche

1. Verfahren zur Herstellung von Sorbinsäureanhydrid, **dadurch gekennzeichnet, dass** man aus einer Mischung enthaltend Sorbinsäureanhydrid und Lösungsmittel das Lösungsmittel mittels eines Verdampfers zumindest teilweise entfernt, wobei der Verdampfer so betrieben wird, dass die Mischung enthaltend Sorbinsäureanhydrid und Lösungsmittel mit einem Inertgasstrom, bevorzugt einem Stickstoffstrom in Kontakt gebracht wird.

2. Verfahren gemäß Anspruch 1, **dadurch gekennzeichnet, dass** der Gehalt an Lösungsmittel im erhaltenen Sorbinsäureanhydrid 1000 ppm oder weniger, bevorzugt 500 ppm oder weniger und besonders bevorzugt 200 ppm oder weniger beträgt.

3. Verfahren gemäß Anspruch 1 oder 2, **dadurch gekennzeichnet, dass** es sich beim Verdampfer um einen Fallfilm- oder Dünnschichtverdampfer, bevorzugt um einen Dünnschichtverdampfer handelt.

4. Verfahren gemäß einem der Ansprüche 1 bis 3, **dadurch gekennzeichnet, dass** das Sorbinsäureanhydrid zu mehr als 96%, bevorzugt zu mehr als 98%, besonders bevorzugt zu mehr als 99% und ganz besonders bevorzugt zu mehr als 99,5 % bezogen auf den Gesamtgehalt an Sorbinsäureanhydrid als all-E-Sorbinsäureanhydrid vorliegt.

5. Verfahren gemäß einem der Ansprüche 1 bis 4, **dadurch gekennzeichnet, dass** die Herstellung der Mischung aus Sorbinsäureanhydrid und Lösungsmittel den Schritt des Inkontaktbringens von ein oder mehreren Alkali- oder Erdalkalisalzen der Sorbinsäure mit Phosgen in einem Lösungsmittel umfasst.

6. Verfahren gemäß Anspruch 5, **dadurch gekennzeichnet, dass** bereits vor dem Einbringen in den Verdampfer ein Teil des Lösungsmittels abgetrennt wird.

7. Verfahren gemäß einem der Ansprüche 1 bis 6, **dadurch gekennzeichnet, dass** nach der Behandlung im Verdampfer das Sorbinsäureanhydrid in einem späteren Schritt durch Kurzwegdestillation weiter aufgereinigt wird.

8. Verfahren gemäß einem der Ansprüche 1 bis 7, **dadurch gekennzeichnet, dass** das Verfahren so geführt wird, dass das Sorbinsäureanhydrid flüssig erhalten und auf einer gekühlten Oberfläche, bevorzugt auf einem Kälteband oder einer gekühlten Schuppenwalze kristallisiert wird.

9. Verfahren gemäß Anspruch 8, **dadurch gekennzeichnet, dass** das Verfahren so geführt wird, dass das kristallisierte Sorbinsäureanhydrid bei einer Temperatur von weniger als 20°C, bevorzugt von weniger als 10 °C und besonders bevorzugt von weniger als 0 °C vermahlen wird.

10. Verfahren gemäß einem der Ansprüche 1 bis 9, **dadurch gekennzeichnet, dass** das Entfernen des Lösungsmittels in Abwesenheit eines Stabilisators erfolgt.

11. Verfahren gemäß einem der Ansprüche 1 bis 9, **dadurch gekennzeichnet, dass** der Mischung enthaltend Sorbinsäureanhydrid und Lösungsmittel ein Stabilisator zugegeben wird

12. Sorbinsäureanhydrid **dadurch gekennzeichnet, dass** der Gehalt an organischem Lösungsmittel 1000 ppm bevorzugt 500 ppm oder weniger und besonders bevorzugt 200 ppm oder weniger beträgt.

13. Sorbinsäureanhydrid gemäß Anspruch 12, **dadurch gekennzeichnet, dass** es zu mehr als 96%, bevorzugt zu mehr als 98%, besonders bevorzugt zu mehr als 99% und ganz besonders bevorzugt zu mehr als 99,5 % bezogen auf den Gesamtgehalt an Sorbinsäureanhydrid als all-E-Sorbinsäureanhydrid vorliegt.

14. Sorbinsäureanhydrid gemäß Anspruch 12 oder 13, **dadurch gekennzeichnet, dass** es zusätzlich einen Stabilisator enthält.

15. Sorbinsäureanhydrid gemäß einem der Ansprüche 12 bis 14 **dadurch gekennzeichnet, dass** es feinteilig in Form von Schuppen, Pellets oder als Pulver vorliegt.

16. Verfahren zur Konservierung von Lebensmitteln, enthaltend zumindest die Schritte gemäß einem der Ansprüche 1 bis 11 sowie den Schritt der Kontaktierung des Lebensmittels mit Sorbinsäureanhydrid.
